# EUROPEAN PATENT SPECIFICATION

(11) **EP 3 196 789 B1**
(45) Date of publication and mention of the grant of the patent: **29.07.2020**
(21) Application number: 17152353.3
(22) Date of filing: 20.01.2017
(51) Int. Cl.: G16H 40/20, G16H 40/40

(54) **MANAGEMENT APPARATUS FOR MEASUREMENT EQUIPMENT**
VERWALTUNGSVORRICHTUNG FÜR MESSAUSRÜSTUNG
APPAREIL DE GESTION D'ÉQUIPEMENT DE MESURE

(30) Priority: 21.01.2016 JP 2016010191
(43) Date of publication of application: 26.07.2017
(73) Proprietor: Horiba, Ltd., Kyoto-city, Kyoto 601-8510 (JP)
(72) Inventor: KAMEZAKI, Ritsuko, Kyoto, 601-8510 (JP)
(74) Representative: Isarpatent

(56) References cited:
- US-A1- 2005 102 167
- US-A1- 2012 182 939
- US-A1- 2013 200 142

## Description

### FIELD OF THE INVENTION

The present invention relates to an apparatus for managing measurement equipment.

### BACKGROUND OF THE INVENTION

When, for example, manufacturers, sales companies and the like that supply various measurement equipments such as blood cell counting device, concentration measuring device and the like (hereinafter such supplying side is to be also referred to as supplier) deliver measurement equipments to various medical institutions (hereinafter to be also referred to as user), it is desirable to not only make delivery but also remotely monitor various kinds of information (operational information) relating to the state of use, measurement sensitivity and the like of the measurement equipments even after delivery and provide users with prompt service and support regarding maintenance, accuracy management and the like.

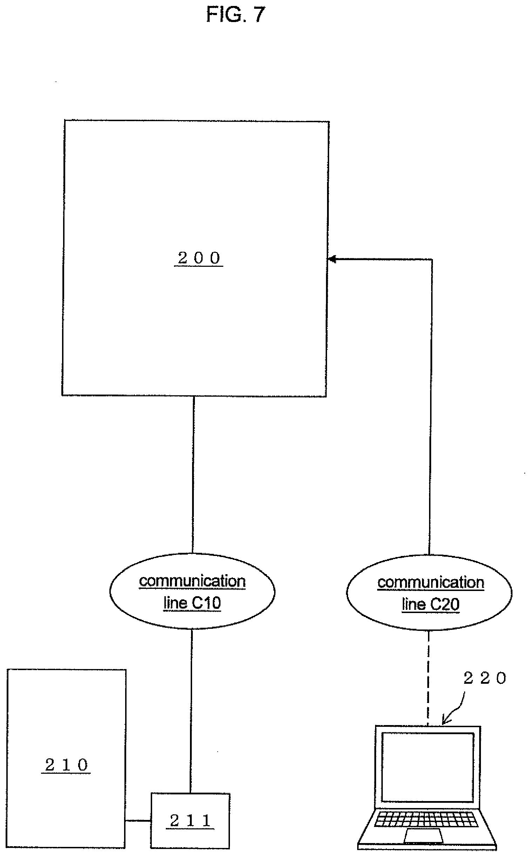

Therefore, as shown in Fig. 7, many suppliers connect measurement equipment 210 delivered to users to a management apparatus (usually a server computer) 200 that the supplier has via a communication terminal 211 and a communication line C10 attached to the measurement equipment 210 to enable data communication, remotely monitor operational information of the measurement equipment 210 by the management apparatus 210, and provide various services and support relating to the measurement equipment based on the monitoring results (e.g., patent document 1 and the like). Patent document 2 relates to a methods and devices providing a wireless communications hub device and services enabling remote access to electronic medical or fitness devices in a manner that simplifies device networking. A wireless communication hub device may include a processor and wireless communication transceivers configured to connect to cellular and/or WiFi networks to access a remote server, and wired and/or wireless local networks for connecting to electronic medical or fitness devices. The wireless communication hub device may plug into a power source, connect to an electronic medical or fitness device, and communicate via a second wireless network with an associated server-based service.

[patent document 1]JP-A-2008-249590
[patent document 2] US 2012/182939 A1

When actually delivering measurement equipment to a user, an operator (generally, a person on the supplier side who delivers measurement equipment to the user and performs connection, setting, etc. of the measurement equipment and the management apparatus but may be a person on the user side) connects the measurement equipment in a communicatable manner to a management apparatus. For the connection, as shown in Fig. 7, it is necessary for the operator to establish data communication between management apparatus 200 and a delivered measurement equipment 210 by confirming an identification number of the measurement equipment 210 (hereinafter the identification number of the measurement equipment is also referred to as measurement equipment ID), accessing the management apparatus 200 by using a communicatable input equipment (portable computer capable of transmitting data to the management apparatus) 220, inputting the measurement equipment ID to the management apparatus 200 through the communicatable input equipment 220, and allowing the management apparatus 200 to recognize the measurement equipment 210.

However, the present inventors examined in detail the actual state of the aforementioned connection and registration operations at the time of delivery of the measurement equipment and found that such operation for connection is a complicated and troublesome work for the operator and there is a possibility that an erroneous measurement equipment ID is registered in the management apparatus due to an input error of the measurement equipment ID.

In view of the aforementioned problem, an object of the present invention is to provide a management apparatus capable of reducing input error of the identification number of the measurement equipment.

### SUMMARY

The invention is defined by the appended claims.
[1] Disclosed is a management apparatus for measurement equipment, the management apparatus comprising,
   a data-accepting part and a data-transmitting-receiving part, wherein
   the data-accepting part accepts, as an input, an identification information of a communication device attached to the measurement equipment to be connected to the management apparatus, and
   the data-transmitting-receiving part communicates with the communication device by using the accepted identification information of the communication device, and obtains identification information of the measurement equipment through the communication device, wherein the identification information is specific to the measurement equipment and held in the measurement equipment.
[2] The management apparatus according to [1], further comprising a user-information-accepting part and a data-processing part,
   the user-information-accepting part accepts, as an input, the information relating to the user of the measurement equipment, and
   the data-processing part generates a character string comprising the information relating to the user and the identification information of the measurement equipment, as a name indicating the measurement equipment.
[3] The management apparatus according to [2], wherein the information relating to the user is a name identifying the user.
[4] The management apparatus according to [1] - [3], further comprising:
   a display-image creating part;
   a display part; and
   an input part,
   wherein
   the display-image creating part operates to display, on the display part, the identification information of one or more communication devices including the identification information of the communication device to be inputted, and the identification information of one or more communication devices displayed on the display part is displayed to be selectable through the input part, and
   the data-accepting part operates to accept the identification information of the communication device selected through the input part as an input of the identification information of the communication device.

### BRIEF DESCRIPTION OF THE DRAWINGS

Fig. 1 is a block diagram showing the constitution of the management apparatus in the embodiments of the present invention. In this drawing, while measurement equipment 100 and a communication device 110 are drawn separately for explanation, the communication device 110 may be a communication part built in the measurement equipment 100. The connection relation of each part in the management apparatus is one embodiment for explanation.
Fig. 2 is a block diagram showing a preferable constitution example of the management apparatus in the embodiments of the present invention. This Figure also shows an embodiment of the communication relation when the measurement equipment delivered to the user is data-communicatably connected to the management apparatus.
Fig. 3 is a flowchart illustrating the flow of processing of the management apparatus in data-communicatably connecting the measurement equipment delivered to the user to the management apparatus in the embodiments of the present invention.
Fig. 4 is a diagram illustrating one embodiment of a preferable constitution for inputting a communication device ID in the embodiments of the present invention, wherein one part of a screen of the display part of the input equipment to be used by the operator is enlarged.
Fig. 5 is a block diagram showing one embodiment of the hierarchical relationship between the supplier and the measurement equipment delivered to each user in the embodiments of the present invention.
Fig. 6 is a diagram illustrating one embodiment of the hierarchical relationship between the supplier and the measurement equipment shown in Fig. 5 as displayed on the screen of the display part.
Fig. 7 is a block diagram showing a conventional constitution of an operation to communicatably connect measurement equipment to a management apparatus.

### DETAILED DESCRIPTION OF THE INVENTION

According to the present invention, when the operator connects the measurement equipment to the management apparatus communicatably, manual input of the measurement equipment ID is not necessary. The management apparatus automatically establishes a communication relation with a communication device by using identification information of the communication device (hereinafter to be also referred to as communication device ID) inputted by the operator, and obtains the measurement equipment ID through the communication device. As a result, the management apparatus and the measurement equipment can communicate through the communication device. Therefore, the labor of the operator concerning the connection at the time of delivery of the measurement equipment is reduced and the problem of input error of the measurement instrument ID is also resolved.

The constitution of the management apparatus of the present invention is explained in detail in the following by referring to Examples. In the following explanation, data-communicatable connection of the measurement equipment to the management apparatus is also simply referred to as connection of the measurement equipment to the management apparatus.

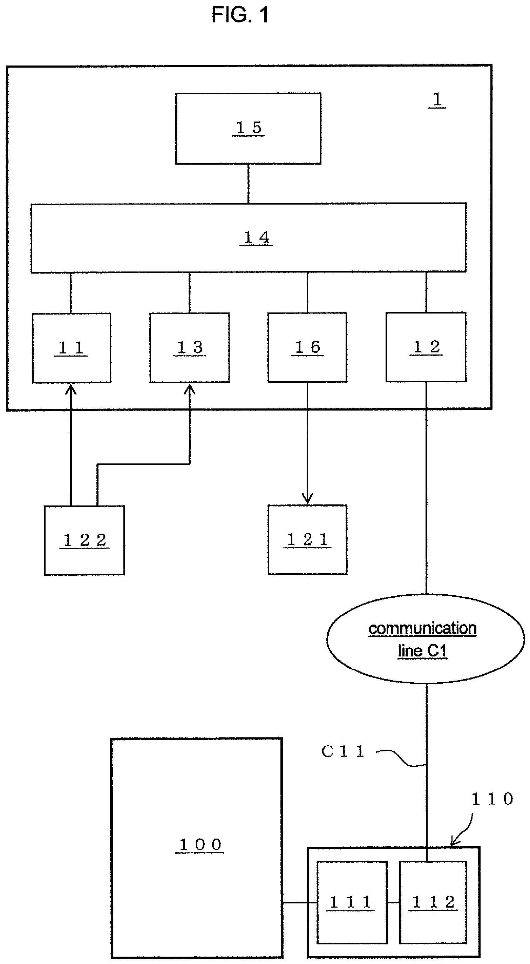

Fig. 1 is a block diagram showing the constitution of the management apparatus in one embodiment of the present invention. Fig. 1 shows one measurement equipment 100 connected to the management apparatus 1 for explanation. A plurality of measurement equipment can be connected to the management apparatus 1, and the number of connections and the number of users are not particularly limited. The management apparatus 1 can remotely monitor the state of each measurement equipment of many connected users, acquire accuracy management information, operational information, maintenance information and the like of each measurement equipment, record and manage them continuously. The supplier side can promptly provide comprehensive maintenance service for measurement equipment of each user based on such information and records.

As shown in Fig. 1, the management apparatus 1 is a device capable of remotely managing a measurement equipment 100, and is constituted to comprise at least a data-accepting part 11 and the a data-transmitting-receiving part 12. In the embodiment of Fig. 1, the management apparatus 1 further comprises, as a preferable embodiment, a user-information-accepting part 13 mentioned below, a data-processing part 14, a storage part 15, and a display-image creating part 16. In the embodiment of Fig. 1, the data-processing part 14 is also a control part that controls data transfer between respective parts of the management apparatus 1 (data-accepting part, data-transmitting-receiving part, user-information-accepting part, storage part and the like). The measurement equipment 100 connected to the management apparatus 1 has identification information (measurement equipment ID) to identify other measurement equipment. The measurement equipment 100 accompanies the communication device 110 intervening the connection with the management apparatus 1, and the communication device 110 has identification information (communication device ID) for communication through a communication line C1.

In a state in which the measurement equipment is merely delivered to the user, the communication path C11 in Fig. 1 is not opened, and the communication device 110 is not connected to the communication line C1. When the measurement equipment 100 is connected to the management apparatus 1 from this state, the data-accepting part 11 operates to accept, as an input, the communication device ID of the communication device 110 attached to the measurement equipment 100 to be connected to the management apparatus 1. In the embodiment of Fig. 1, the communication device ID is inputted from the input part 122 by an operator (i.e., person on the supplier side or person on the user side). The input part for actually inputting the communication device ID is mentioned below. In addition, a preferable embodiment using the display part and the input part for the operator to input the communication device ID with ease and with less error is also mentioned below.

The data-transmitting-receiving part 12 uses the accepted communication device ID to first establish communication relation with communication device 110 through the communication line C1. Then, the data-transmitting-receiving part 12 obtains identification information (measurement equipment ID) of the measurement equipment 100 through communication with the communication device 110, and operates to establish communication relation (data-communicatable relation) with the measurement equipment 100 via the communication device 110. That the data-transmitting-receiving part establishes communication relation with the measurement equipment via the communication device means that the data-transmitting-receiving part and the measurement equipment are in a relation enabling communication via the communication device, for example, a relation in which a message or the like is transmitted to a measurement equipment recognized by the data-transmitting-receiving part, and a message, information data and the like are received as a reply thereof from the measurement equipment, and a relation in which the measurement equipment unilaterally transmits a message, information data or the like to the data-transmitting-receiving part, and the data-transmitting-receiving part identifies the measurement equipment and receives the message, information data, and the like. When the communication relation between the data-transmitting-receiving part and the measurement equipment is established, an operation to connect the measurement equipment 100 to the management apparatus 1 is completed. As shown in Fig. 1, it is preferable to provide, in the management apparatus, a storage part 15 for holding the communication device ID (which may be a telephone number or the like associated with the communication device ID) to be inputted and the measurement equipment ID to be obtained. In the storage part 15, it is preferable that the communication device ID and the measurement equipment ID be held in association with each other. Information on the user mentioned below may be stored in the storage part 15, and it is preferable that the information on the user, and the communication device ID and the measuring equipment ID be held in association with each other.

As the mentioned above, when the unconnected measurement equipment 100 is newly connected to the management apparatus, the operator inputs at least the communication device ID to the management apparatus, whereby the measurement apparatus 100 can be connected to the management apparatus 1. As a result, an input error of the measurement equipment ID does not occur. Even when the input of the communication device ID is mistaken, the input error can be known immediately since communication with the communication device is not performed.

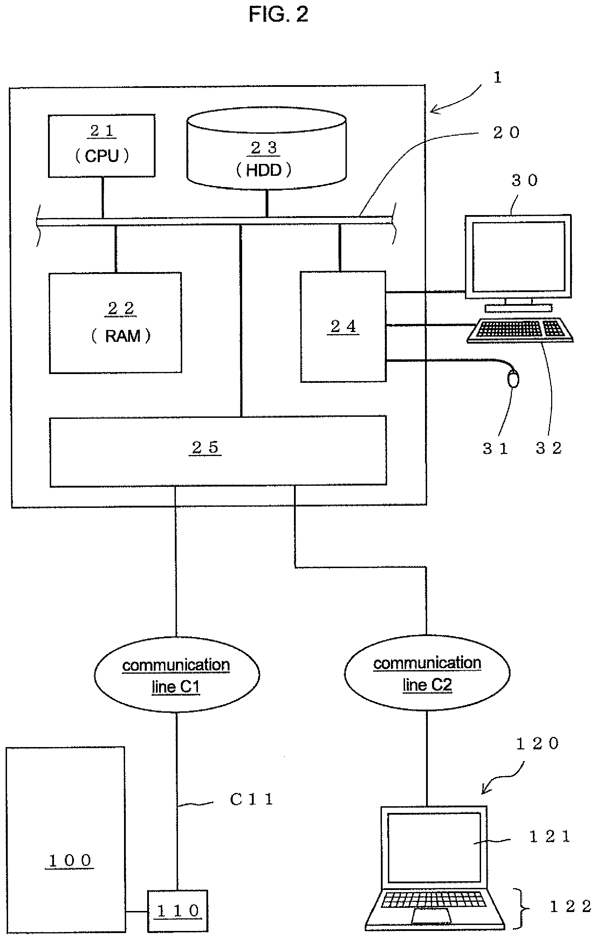

Each part constituting the management apparatus (data-accepting part 11, data-transmitting-receiving part 12, user-information-accepting part 13, data-processing part 14, storage part 15, display-image creating part 16 and the like) may be constituted by a combination of electronic circuit, electric circuit, separate processing unit, communication equipment and the like. In a preferable embodiment, each of these parts are constituted by a computer and using a program executed in the computer as shown in Fig. 2.

A preferable embodiment and an effect thereof are explained in the following by referring to an Example when the management apparatus is constituted by a computer. The constitution of each part described below can be performed instead of a computer by partly or entirely combining an electronic circuit, an electric circuit, an independent processing device, a communication device and the like.

Fig. 2 is a block diagram showing a preferable embodiment of the management apparatus, and shows an embodiment wherein the management apparatus is constituted by a computer. In the embodiment of Fig. 2, the management apparatus 1 may be a server computer for remotely managing measurement equipment 100 delivered to users for maintenance services.

The basic architecture itself of the management apparatus 1 as a computer shown in Fig. 2 may be the same as the conventionally known computer. As shown in Fig. 2, a computer as the management apparatus 1 comprises a constitution wherein a central processing unit (CPU) 21 and a main memory (RAM in the embodiment of Fig. 2) 22 are connected by a data bus 20, preferably a hard disk drive (HDD) 23 is connected and gives CPU 21 a storage area with large capacity. The hard disc drive may be appropriately replaced by other storage device such as solid-state drive and the like. In addition, it may be connected to an external storage device or external computer so that large amounts of data continuously obtained from many measurement equipments can be stored.

In the embodiment of Fig. 2, display device 30, mouse 31 and keyboard 32, as the display part and input part attached to the computer itself, are connected to a data bus 20 via an interface 24. These display part and input part can also be used, for example, by an administrator to change or update the contents and the like of the program executed by the management apparatus as necessary, and can also be used for the analysis of the records accumulated in the management apparatus.

When the management apparatus is a computer, the data-accepting part 11, data-transmitting-receiving part 12, user-information-accepting part 13, data-processing part 14 and the like shown in Fig. 1 are the parts where the computer works according to the program executed in the computer (the storage part 15 may include main memory). In other words, the program is constituted to cause a computer as the management apparatus to function as data-accepting part 11, data-transmitting-receiving part 12, user-information-accepting part 13, data-processing part 14, the below-mentioned display-image creating part 16 and the like. Each of the above-mentioned functions (operation contents) achieved by executing the program includes not only processing performed by CPU 21 and main memory 22, but also the functions achieved by using each hardware constituting the management apparatus and external hardware, under the instruction by the program. Furthermore, by the control of the program, the function of each part may be executed in an external device connected to the management apparatus, and function of each part may be executed by the cooperation of the management apparatus and the external device.

In the embodiment of Fig. 2, the data-accepting part 11 and data-transmitting-receiving part 12 of Fig. 1 are parts that perform data communication with an external device, and can be constituted using at least CPU 21, main memory 22 and interface 25 such that data communication is possible with input equipment (having input part 122, display part 121) 120, communication device 110, measurement equipment 100 and the like.

To explain the communication device ID, as an input, to be accepted by the data-accepting part 11, the measurement equipment 100 and communication device 110 shown in Fig. 1 are explained.

While the measurement equipment 100 to be connected to the management apparatus is not particularly limited, equipment by which service of remote management such as continuous monitoring and the like is favorably received affords remarkable usefulness of the present invention. Examples of such measurement equipment include equipment that can measure and analyze items relating to various tests (hematological examination, biochemical examination, immunological examination, genetic examination) using a specimen (blood, urine, feces, cell and the like) as a test target. For example, blood cell counting device, blood analysis device, concentration measuring device, immunity measuring device, device for genetic analysis and the like can be mentioned.

The measurement equipment 100 to be connected to the management apparatus has an output port (wiring output terminal and/or wireless transmitting and receiving part and the like), such that a supplier can output information data for providing maintenance service to users. Many measurement equipments in recent years have a computer built in as a control part and an output port such as an interface for data communication with the outside, even when they are compact instruments such as desktop type and palm size (palmtop type) and the like, and can output the operational information as data to the external device. Therefore, they are preferably connected to the management apparatus. The storage part or interface in the measurement equipment 100 holds, in the communication with the external device such as the management apparatus and the like, identification information (measurement equipment ID) for identification of the measurement equipment itself and other measurement equipment by the external device. The management apparatus identifies each measurement equipment based on the measurement equipment ID of each measurement equipment, and accumulates information data showing operation state for each measurement equipment.

In the embodiment of Fig. 1, Fig. 2, the communication device 110 attached to the measurement equipment 100 is drawn to be a communication device other than the measurement equipment 100 for easy understanding as a block diagram. However, it may be a communication device part built in the measurement equipment or a separate communication device data-communicatably connected to the measurement equipment. It is preferable that the communication device 110 be constituted to be able to mediate communication between the management apparatus 1 and the measurement equipment 100 by not only performing data communication with the management apparatus 1, but also with a control part (not shown) in the measurement equipment 100.

The communication device 110 acquires a measurement equipment ID from the control part of the measurement equipment 100 in response to the request of the management apparatus 1, transmits the measurement equipment ID to the management apparatus, and establishes the data communication between the measurement equipment and the management apparatus.

The communication device 110 has an identification information (communication device ID) for the communication with the management apparatus through the communication line C1. When the communication device is built in the measurement equipment, the communication device ID is displayed or notified so that the operator can know by, for example, being described on a label affixed to the measurement equipment or an attached document. Also, even when the communication device is a communication device different from the measurement equipment, similarly, the communication device ID is displayed or notified so that the operator can know by, for example, being described on a label affixed to the measurement equipment or an attached document. The operator inputs the communication device ID to the management apparatus, by which the management apparatus can access a communication device having the communication device ID.

A communication line C1 between communication device 110 and the management apparatus may be wired or wireless, or may be a combination thereof. Examples of the communication line include any available communication lines such as wired or wireless local area network (LAN), Internet, telephone line, mobile broadband (wireless broadband) and the like. These can be used alone or in combination with any combination of these. A local section such as a terminal part of a communication line and the like may include a communication path conforming to an interface such as Bluetooth (registered trade mark), USB, RS-232C and the like.

The communication device 110 is constituted to enable data communication through the communication line to be used, and a modem (modulation-demodulation device), a splitter, an optical line termination device, a router, a digital/analog converter and the like may be further attached as necessary.

The communication device ID only needs to be information that enables identification of the communication device. When the communication device is a device that communicates via LAN or Internet, it may be an IP address obtained for the communication device by contracting with a provider. When the communication device is a device that communicates via a telephone line or mobile broadband, it is, for example, given identification information or a telephone number obtained for the communication device by contracting with a telecommunications carrier. The telephone number may be held in the management apparatus in association with the aforementioned given identification information. In this case, for example, when the given identification information is inputted as a communication device ID, the management apparatus may start access to the communication device by using the telephone number associated with the communication device ID.

The communication path C11 on the communication device 110 side is preferably wireless to place measurement equipment without introducing wiring in the medical field, and to eliminate the trouble of wiring connection. In the wireless, a communication using mobile broadband can be mentioned as a preferred embodiment in which the operator can more easily establish connection with the management apparatus. In the following, an example configuration of a communication device, example inputting of a communication device ID, and an example connection procedure are explained by reference to an embodiment using a mobile broadband as a communication line. When other communication line is utilized, similarly, the data communication between measurement equipment and the management apparatus is automatically established when an operator inputs at least a communication device ID.

In the embodiments shown in Fig. 1, Fig. 2, the communication device 110 and the measurement equipment 100 are devices different from each other. As mentioned above, however, they may form an integrated device. In the embodiment shown in Fig. 1, the communication device 110 has a control part 111 and a communication part 112, and the control part 111 is connected to a control part (not shown) of the measurement equipment 100 to allow for data communication via each interface (not shown). When the communication device 110 and the measurement equipment 100 form an integrated device, the control part 111 may be a control part common to the both.

The control part 111 of the communication device 110 is constituted such that it performs data communication with the measurement equipment 100 in response to the request from the management apparatus 1, obtains a measurement equipment ID from the measurement equipment 100, and transmits the measurement equipment ID to the management apparatus 1 via the communication part 112. Such control part 111 may be a computer (one chip microcomputer, one board microcomputer and the like), may be constructed with hardware such as electronic circuit, electric circuit and the like, and one constituted to respond to the command of the management apparatus 1 and enabling data communication with the measurement equipment is preferably used.

In the embodiment shown in Fig. 1, the communication part 112 of the communication device 110 is a device capable of data communication through a mobile broadband (e.g., 3G line in portable telephone and the like) as a communication line C1. One of the plural SIM (Subscriber Identity Module) cards obtained by a supplier by contracting with a telecommunications carrier is attached to the communication part 112, which enables data communication through a mobile broadband. A telephone number unique to the SIM card is given from a telecommunications carrier. In the embodiment shown in Fig. 1, the telephone number is the communication device ID. The telephone number is described on the label affixed to the casing of the communication device 110, so that the operator can confirm the telephone number.

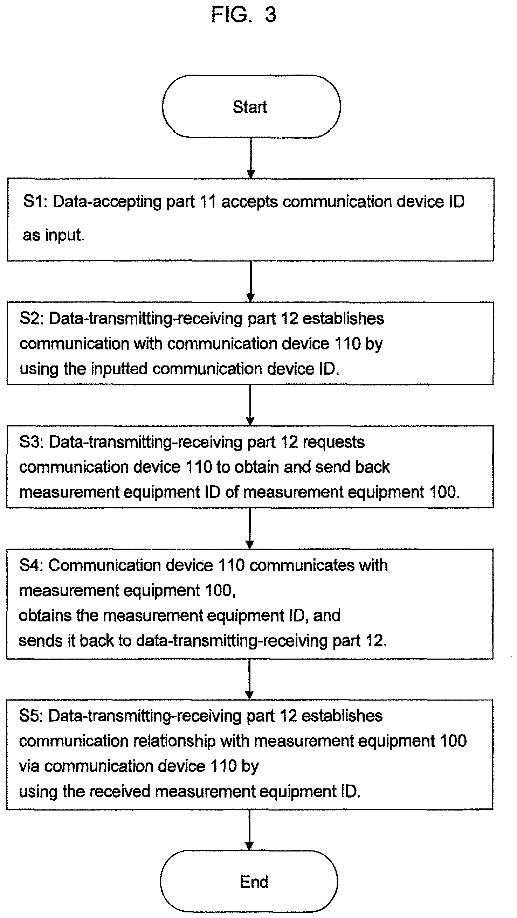

The procedure for connecting the measurement equipment 100 to the management apparatus 1 in the embodiments shown in Fig. 1, Fig. 2 is explained along the flowchart shown in Fig. 3.

First, in step S1, the data-accepting part 11 of the management apparatus 1 accepts the communication device ID of the communication device 110 as an input from the input part 122. In this embodiment, an operator who delivered measurement equipment to a user remotely inputs a telephone number as the communication device ID to the management apparatus from the input part of the terminal which is data-communicatable with the management apparatus. The embodiments of the input terminal and a preferable input are mentioned below.

Then, in step S2, the data-transmitting-receiving part 12 calls the communication device 110 having a telephone number as the inputted communication device ID and establishes communication with the communication part 112 of the communication device 110 through the mobile broadband line.

In step S3, the data-transmitting-receiving part 12 requests the control part 111 via the communication part 112 to acquire and send back the measurement equipment ID of the measurement equipment 100.

In step S4, the control part 111 of the communication device 110 communicates with the measurement equipment 100, obtains the measurement equipment ID, and sends back the measurement equipment ID to the data-transmitting-receiving part 12.

In step S5, the data-transmitting-receiving part 12 of the management apparatus 1 stores (registers) the measurement equipment ID in the storage part 15 to individually manage the measurement equipment 100, and establishes a communication relationship with the measurement equipment via the communication device.

In the above steps, since the operator does not need to input the measurement equipment ID, an input error of the measurement equipment ID does not occur and the connection between the measurement equipment and the management apparatus can be performed smoothly.

When the measurement equipment and the communication device are in a one-to-one relationship, since the connection relationship is simple, the connection operation is easy to understand and easy for the operator and the like. However, a plurality of measurement equipment may be connected to one communication device.

The data-accepting part 11 accepts the communication device ID from an input part for inputting the communication device ID. The input part for inputting the communication device ID is a device for inputting data to the data-accepting part of the management apparatus and may be an input part (mouse 31, keyboard 32, etc. in Fig. 2) attached to the management apparatus. Alternatively, it may be an input part (e.g., input part such as mouse, keyboard, touch panel etc. attached to the communication terminal device 120 etc. in Fig. 2) data-communicatably connected to the management apparatus. It is preferable to prepare the input parts in a number necessary for enabling a plurality of operators to use them separately.

In the embodiment shown in Fig. 2, communication device ID is inputted from the input part 122 of the communication terminal device 120 through the communication line C2 and the interface 25. In Fig. 2, only one input equipment 120 is drawn for explanation. To simply and certainly input communication device ID, the input equipment 120 is preferably a portable type terminal computer having a display part 121 and an input part (keyboard, touch-pad, touch panel and the like) 122, as shown in Fig. 2. For example, the portable type terminal computer as an input equipment 120 is preferably a laptop computer (notebook computer) shown in Fig. 2, a tablet computer, a computer-loaded type portable telephone (portable information terminal or smartphone) or the like.

The communication line C2 between the management apparatus 1 and input equipment (having input part 122) 120 may be wired or wireless, and an operator can preferably use various communication lines such as a line including a wireless line such as a wireless LAN and Wi-Fi (registered trade mark) and the like and the Internet, a mobile broadband line and the like. The communication line C2 and the above-mentioned communication line C1 may be lines of the same type.

When a portable type terminal computer as shown in Fig. 2 is used as an input part for inputting the communication device ID to the management apparatus 1, the program for inputting the communication device ID may be a program to be individually executed (a program that accepts the communication device ID and transmits it to the management apparatus) in each terminal computer. However, in a preferable embodiment, each terminal computer utilizes an input program to be executed in the management apparatus (computer). For example, on the Internet, a program executed in a web server is configured such that characters and symbols from each terminal computer can be inputted to an input window displayed on a screen of a website, and the display part and the input part cooperate to constitute an input function. Similarly, a constitution is preferable in which the input display image and the input window are displayed on the display part (display device) 121 of each terminal computer by the program in the management apparatus side (display-image creating part 16) and the input screen is operated and the communication device ID and other accessorial input items are inputted to the data-accepting part 11 by the input part (keyboard, click button, touch panel and the like) 122 of each terminal computer 120. In the embodiment of Fig. 2, the display part 121 is a display device attached to the input equipment 120 data-communicatable with the management apparatus. However, it is not limited to such embodiment and may be a device that displays the data outputted from the management apparatus. In the embodiment of Fig. 2, the input part 122 is an input device attached to the input equipment 120 data-communicatable with the management apparatus. However, it is not limited to such embodiment and may be a device that inputs data to a data-accepting part of the management apparatus as mentioned above.

As the program on the management apparatus side, various conventionally known programming languages such as Java (registered trade mark) and the like, which are used for creating a website, can be used, and a descriptive language such as HTML may also be used.

While the communication device ID may be manually inputted by the operator, an input error may occur by manual input. Therefore, in a preferable embodiment of the present invention, the management apparatus 1 further comprises the display-image creating part 16, and the display-image creating part 16 displays one or more communication device IDs containing the communication device ID to be inputted on the display part 121 of the input equipment (terminal computer and the like) to be used by the operator to perform selection. The display-image creating part 16 creates data to be displayed, and the data-output part (or output data creating part) may output the data to the display part 121. The image to be displayed is not limited and may be an image such as character, symbol and the like. The communication device ID displayed in the display part 121 is selectable through the input part (mouse, touch pad, keyboard, touch panel and the like of input equipment) 122 (display part 121 and input part 122 are preferably constituted to enable an operator to input communication device ID, and the display part preferably displays pointer and cursor that move according to the instructions from the input part).

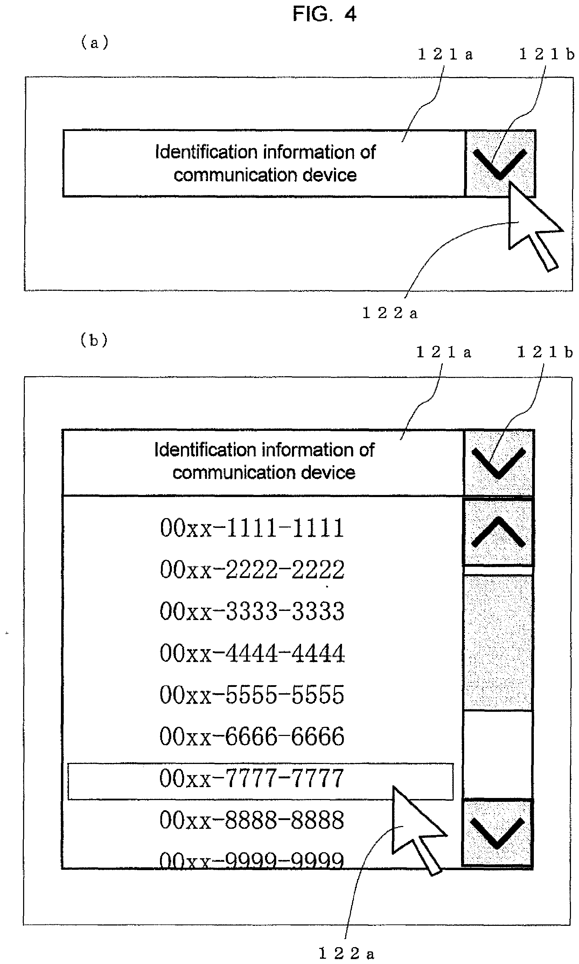

For example, when the above-mentioned mobile broadband is utilized as a communication line and the communication device ID is a telephone number given by the above-mentioned SIM card, the display-image creating part 16 displays, as an image, an input window 121a for inputting "Identification information of communication device" on the display part 121, as shown in Fig. 4(a). An arrow 121b is selected through a pointer 122a on the screen operated on by the input part 122. When the input operation (touching the area of the selected communication device ID in the case of clicking a physical button or touching a touch panel) is performed, the display-image creating part displays currently available communication device IDs as a pull-down menu as shown in Fig. 4(b). As for the constitution and operation of the pull-down menu itself, known technology can be referred to. The operator selects, with pointer 122a at the site of the delivery of the measurement equipment (may be at a sales company, a factory or the like on the supplier side), one matching with the communication device ID displayed on the casing of the communication device ("00xx-7777-7777" in the embodiment of Fig. 4(b)), and performs an input operation to input the communication device ID. The data-accepting part accepts the inputted communication device ID, and the management apparatus automatically achieves connection with the measurement equipment as mentioned above. A constitution requiring two-step input may be employed, in which a start button (confirmation button) to start communication with the communication device is further displayed on the screen of the display part and, after confirmation of the operator's inputting of the correct communication device ID, the start button is pressed to start communication.

In the embodiment of Fig. 4, while a pull-down menu was used as a display embodiment of communication device ID, all communication device IDs may be displayed by arranging them as a table on the screen, and a display design and an input method (such as scroll and various inputs using a touch panel) for making it easier for the operator to select a communication device ID may be adopted as appropriate.

When many communication device IDs acquired in advance by the supplier side are deleted from the aforementioned display at the stage when connection with the management apparatus is established, erroneous selection of the communication device ID is reduced. The communication device 110 shown in Fig. 1, Fig. 2 is in an uncommunicatable state without supply of an electric power (i.e., only those installed by the user can communicate). Therefore, even when the communication device ID is erroneously selected, communication is not performed and no error or confusion occurs.

When the measurement equipment delivered to the user is connected to the management apparatus, it is preferable that the management apparatus accept not only the measurement equipment ID but also information on the user (such as medical institution and the like) where the measurement equipment is installed and hold the information in the storage unit 15 in association with the measurement equipment ID. In the preferable embodiment of the present invention, as shown in Fig. 1, the management apparatus futher comprises a user-information-accepting part 13 and a data-processing part 14. The user-information-accepting part 13 operates to accept the aforementioned information relating to the user as an input. The information relating to the user includes, for example, character string, symbol and the like with which the operator can identify the user, such as user name, abbreviated name of user, initial letter and the like. The data-processing part 14 generates, as the name of the measurement equipment, having the information on the user accepted as input and the measurement equipment ID of the delivered measurement equipment, a character string, stores it in the storage part 15, and displays it on the display part 121.

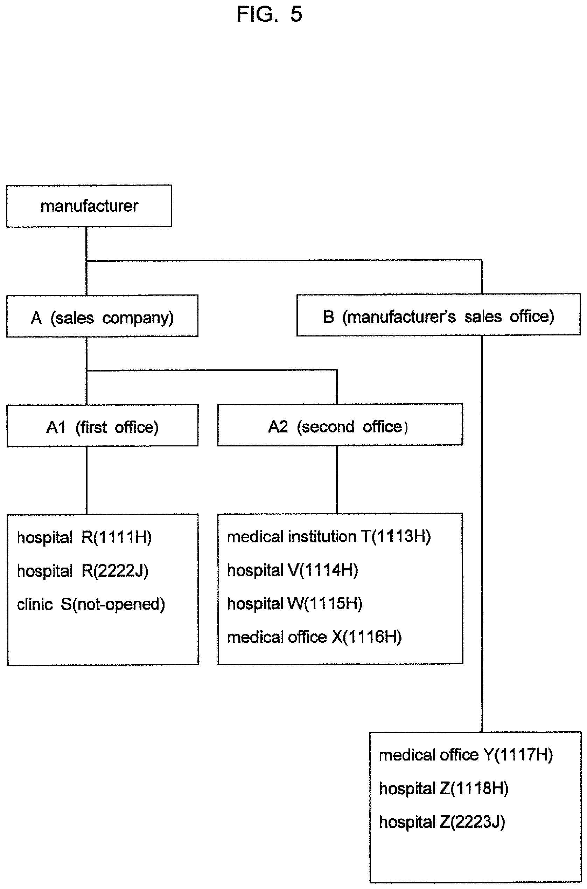

Fig. 5 is a block diagram showing one embodiment of the hierarchical relationship between the supplier and the measurement equipment delivered to each user in the embodiments of the present invention. In the hierarchical embodiment of Fig. 5, for explanation, measurement equipment is sold to a user through a sales company or a sales office of a manufacturer. It may be direct sales from a factory of the manufacturer, or only by a sales company. The uppermost part of the relational diagram may be not only a manufacturer but also a medical equipment sales company and the like.

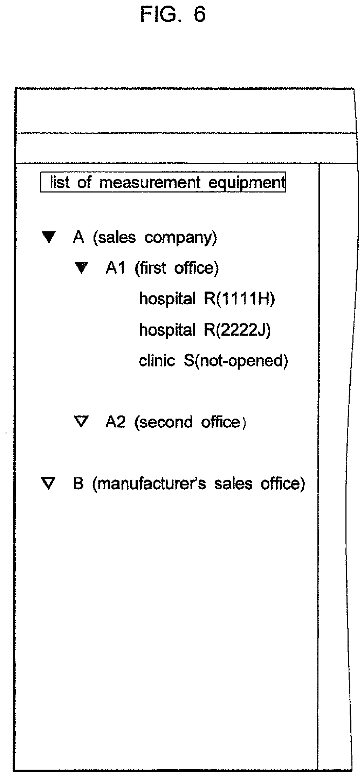

Fig. 6 is a diagram illustrating one embodiment of the hierarchical relationship between the supplier and the measurement equipment shown in Fig. 5 as displayed on the screen of the display part to facilitate understanding of the operator. A hierarchical association between the measurement equipment delivered to the user and the supplier is displayed in a tree-shape in the pane (divided display region) on the left side, as shown in Fig. 6, on the screen of the display part (e.g., display device of input equipment 120 to be used by the operator) 121. In the embodiment of Fig. 6, "A (sales company)" is selected by the input part 122, due to which "A1 (first office)" and "A2 (second office)" are developed and displayed thereunder, "A1 (first office)" is selected by the input part 122, and three measurement equipments are developed and displayed thereunder. A display of "clinic S(not-opened)" is mentioned below. While "A2 (second office)" and "B (manufacturer's sales office)" also have the measurement equipment delivered to the user as in Fig. 5, such measurement equipment is not developed in the embodiment of Fig. 6.

As shown in Fig. 6, by displaying hierarchical-like association on the screen of the display part, the operator and the technical person on the supplier side and the like can more clearly manage the user and the individual measurement equipment in relation to each other and provide an error-free maintenance service to the user.

An embodiment of an operation to relate the information relating to the user and the measurement equipment ID is explained below.

In the embodiments of Fig. 5, Fig. 6, measurement equipment (identification information 1111H) was already delivered from the first office A1 of the sales company A to hospital R, and the connection with the management apparatus has been completed. For the connection, a user-information-accepting part 13 requests, through the above-mentioned display-image creating part 16 and display part 121, the operator to input the information (name in this embodiment) of the delivery destination (user). When the operator inputs the user name "hospital R", the user-information-accepting part 13 accepts the input, and the data-processing part 14 generates a character string "hospital R(1111H)" having "hospital R" and the measurement equipment ID "1111H" obtained from the measurement equipment, as the name of the measurement equipment. Then, as shown in Fig. 6, the character string "hospital R(1111H)" is displayed to belong to the first office A1. The character string "hospital R(1111H)" is one embodiment, and any character string appropriately containing the information relating to the user and the measurement equipment ID, such as hospital R-1111H, hospitalR1111H and the like, can be used. A character string containing a measurement equipment ID after a user name is preferable since the association between the user and the measurement equipment can be more intuitively grasped.

Even when plural measurement equipments are delivered to the same user, an embodiment containing a user name for each name of the measurement equipment, such as "hospital R(1111H)" and "hospital R(2222J)" is easily understood and preferable as shown in embodiments of Fig. 5, Fig. 6.

When the measurement equipment is connected to the management apparatus, it is preferable to request the operator to input information about the user prior to inputting the communication device ID (or in the same input screen simultaneously with the input of the communication device ID). For example, as shown in Fig. 6, when measurement equipment is delivered from the first office A1 of the sales company A to clinic S, the user-information-accepting part 13 requests the input equipment of the operator to input the name of the user. When the operator inputs "clinic S", the user-information-accepting part 13 accepts the character string "clinic S", the data-processing part 14 generates a character string such as "clinic S(not-opened)" and the like as a name for a newly delivered measurement equipment, and "clinic S(not-opened)" is displayed on the display part. Then, when the operator inputs a communication device ID according to the above-mentioned step, and the management apparatus automatically establishes connection with the measurement equipment, the data-processing part generates a character string including the user name "clinic S" and the measurement equipment ID (e.g., 1112H), and
"clinic S(not-opened)" on the display part changes to "clinic S(1112H)". With this input order, the operator can confirm that the connection between the management apparatus and the measurement equipment has been established, and can definitely and easily complete the association between the user and the measurement equipment.

In a preferable embodiment, the management apparatus may be configured to identify the product information of the measurement equipment having the measurement equipment ID based on the acquired measurement equipment ID, and to display the product information on the screen of the above-mentioned display part.

Examples of the product information of the measurement equipment include model; device type; item number; product number; production date; history of services; history of inquiries; various items to be added as necessary and the like.

For the identification of the product information of the measurement equipment based on the measurement equipment ID, for example, a table in which the measurement equipment ID and the product information are related may be held in the above-mentioned storage part or the like and the product information corresponding to the measurement equipment ID may be obtained from the table.

By displaying the product information when acquiring the measurement equipment ID, statistical analysis and the like by product information, and the like can be performed in addition to the improvement of the user support, and more accurate user support can be performed.

In the above-mentioned embodiments, the communication devices were set up by users and enabled to communicate by being supplied with electric power. In a modified embodiment, a switching part such as a switch and the like for switching the communication function to ON/OFF may be provided in the communication device, and the communication function may be switched ON when the communication device is installed for the user.

In the above-mentioned embodiments, the data-transmitting-receiving part 12 first called the communication device 110 through the telephone line. In a modified embodiment, the data-transmitting-receiving part 12 first transmits data to the communication device 110 through a short message service (SMS) and the like, after which the communication with the communication part 112 of the communication device may be established.

In the above-mentioned embodiments, SIM card was purchased from a telecommunications carrier. In a modified embodiment, the SIM card to be attached to the communication device 110 is purchased from a telecommunications carrier, and a contract may be further concluded with a telecommunications carrier to bring the communication to a dormant state or a line unopened state. In this way, it is possible to reduce the cost relating to the holding of the SIM card from the purchase of the SIM card to the delivery to the user. The pause state of the communication can be released by correspondence or communication with the telecommunications carrier (communication from the management apparatus to the line connection device of the telecommunications carrier including a request to open the line), when the communication device attached with the SIM card is delivered to the user together with the measurement equipment and the measurement equipment is connected to the management apparatus.

In the above-mentioned embodiments, the measurement equipment used in the medical field is cited as an embodiment of the management target of the management apparatus. However, the measurement equipment to be the management target of the management apparatus may be measurement equipment (including measurement equipment and analysis device) in not only the medical field but also any industrial field. For example, in the field of environmental measurement, the quality of river water, air pollution and the like are continuously measured by measurement equipment. The management apparatus can perform such continuous measurement and can preferably manage measurement equipment that requires continuous management for that measurement.

The constitution wherein the management apparatus of the present invention and measurement equipment are data-communicatably connected can also be said a management system for measurement equipment, which comprises the management apparatus and measurement equipment (further having an input part to be used by the operator). In addition, the state where an input part for inputting the communication device ID is data-communicatably connected to the management apparatus of the present invention can also be said a connection system for newly connecting the measurement equipment having a management apparatus and an input part.

While the management apparatus is connected to the measurement equipment such that the operational information of the measurement equipment is remotely monitored, the connection aims at services and support for the user of the measurement equipment itself. Therefore, it is preferable to prevent unintentional transmission, from the measurement equipment to the management apparatus, of such information (e.g., information that can identify individual person such as specimen ID and the like) relating to the specimen handled by the measurement equipment on the user side.

From such aspect, in a preferred embodiment of the management apparatus (or a management system including the management apparatus and the measurement equipment), a communication-setting part capable of setting one or both of the management apparatus and the measurement equipment enabling or disabling communication of the specimen information in them is preferably provided. Particularly, it is more secure and preferable to provide a communication-setting part to both the measurement equipment and the management apparatus because, even when the measurement equipment is set to enable communication of specimen information, the specimen information is not received by the management apparatus unless the management apparatus is set to enable communication of specimen information. Furthermore, it is a more preferable embodiment from the aspect of security to provide a constitution of the communication-setting part in the management apparatus, which prevents any one other than a person having specific authority on the supplier side from changing the setting of the communication-setting part to allow communication.

According the present invention, an input error of a measurement equipment ID can be reduced when the measurement equipment is delivered to the user and the measurement equipment is data-communicatably connected to the management apparatus such as a server computer and the like, and the connection can be easily completed.

The operator can more certainly identify and manage each measurement equipment by using a character string having the information on the user and the identification information on the delivered measurement equipment as the name of each measurement equipment.

## Claims

1. A management apparatus (1) for measurement equipment (100), the management apparatus (1) comprising,
a data-accepting part (11) and a data-transmitting-receiving part (12), wherein
the data-accepting part (11) accepts, as an input, an identification information of a communication device (110) attached to the measurement equipment (100) to be connected to the management apparatus (1), and
the data-transmitting-receiving part (12) communicates with the communication device (110) by using the accepted identification information of the communication device (110), and obtains identification information of the measurement equipment (100) through the communication device (110), wherein the identification information is specific to the measurement equipment and held in the measurement equipment,
the management apparatus (1) further comprising a user-information-accepting part (13) and a storage part (15), **characterized in that**
the user-information-accepting part (13) accepts, as an input, information relating to a user of the measurement equipment (100, and
the storage part (15) holds the information relating to the user and the identification information of the communication device (110), and the identification information of the measurement equipment (100) in association with each other.

2. The management apparatus (1) according to claim 1, further comprising a data-processing part (14),
the data-processing part (14) generates a character string comprising the information relating to the user and the identification information of the measurement equipment (100), as a name indicating the measurement equipment (100).

3. The management apparatus (1) according to claim 2, wherein the information relating to the user is a name identifying the user.

4. The management apparatus (1) according to any of claims 1 to 3, further comprising:
a display-image creating part (16);
a display part (121); and
an input part (122),
wherein
the display-image creating part (16) operates to display, on the display part (121), the identification information of one or more communication devices (110) including the identification information of the communication device (110) to be inputted, and the identification information of one or more communication devices (110) displayed on the display part (121) is displayed to be selectable through the input part (122), and
the data-accepting part (11) operates to accept the identification information of the communication device (110) selected through the input part (122) as an input of the identification information of the communication device (110).

## Patentansprüche

1. Verwaltungsvorrichtung (1) für Messausrüstung (100), wobei die Verwaltungsvorrichtung (1) einen Datenannahmeteil (11) und einen Datenübertragungs- und -empfangsteil (12) umfasst, wobei
der Datenannahmeteil (11) als Eingabe Identifikationsinformationen einer Kommunikationseinrichtung (110) annimmt, die an die mit der Verwaltungsvorrichtung (1) zu verbindende Messausrüstung (100) angeschlossen ist, und
der Datenübertragungs- und -empfangsteil (12) mit der Kommunikationseinrichtung (110) unter Verwendung der angenommenen Identifikationsinformationen der Kommunikationseinrichtung (110) kommuniziert und durch die Kommunikationseinrichtung (110) Identifikationsinformationen der Messausrüstung (100) bezieht, wobei die Identifikationsinformationen spezifisch für die Messausrüstung sind und in der Messausrüstung gespeichert sind,
wobei die Verwaltungsvorrichtung (1) ferner einen Nutzerinformations-Annahmeteil (13) und einen Speicherteil (15) umfasst,
**dadurch gekennzeichnet, dass**
der Nutzerinformations-Annahmeteil (13) einen Nutzer der Messausrüstung (100) betreffende Informationen als Eingabe annimmt und
der Speicherteil (15) die den Nutzer betreffenden Informationen und die Identifikationsinformationen der Kommunikationseinrichtung (110) und die Identifikationsinformationen der Messausrüstung (100) in Verbindung miteinander speichert.

2. Verwaltungsvorrichtung (1) nach Anspruch 1, ferner umfassend einen Datenverarbeitungsteil (14),
wobei der Datenverarbeitungsteil (14) eine die den Nutzer betreffenden Informationen und die Identifikationsinformationen der Messausrüstung (100) umfassende Zeichenfolge als einen die Messausrüstung (100) anzeigenden Namen erzeugt.

3. Verwaltungsvorrichtung (1) nach Anspruch 2, wobei es sich bei den den Nutzer betreffenden Informationen um einen den Nutzer identifizierenden Namen handelt.

4. Verwaltungsvorrichtung (1) nach einem der Ansprüche 1 bis 3, ferner umfassend:
einen Anzeigebild-Erstellungsteil (16),
einen Anzeigeteil (121) und
einen Eingabeteil (122),
wobei
der Anzeigebild-Erstellungsteil (16) so arbeitet, dass er auf dem Anzeigeteil (121) die Identifikationsinformationen einer oder mehrerer Kommunikationseinrichtungen (110) einschließlich der einzugebenden Identifikationsinformationen der Kommunikationseinrichtung (110) anzeigt und die auf dem Anzeigeteil (121) angezeigten Identifikationsinformationen einer oder mehrerer Kommunikationseinrichtungen (110) so angezeigt werden,
dass sie durch den Eingabeteil (122) auswählbar sind, und
der Datenannahmeteil (11) so arbeitet, dass er die durch den Eingabeteil (122) ausgewählten Identifikationsinformationen der Kommunikationseinrichtung (110) als Eingabe der Identifikationsinformationen der Kommunikationseinrichtung (110) annimmt.

## Revendications

1. Appareil de gestion (1) d'équipement de mesure (100), l'appareil de gestion (1) comprenant :
une partie d'acceptation de données (11) et une partie de transmission-réception de données (12), dans lequel
la partie d'acceptation de données (11) accepte, en tant qu'entrée, une information d'identification d'un dispositif de communication (110) attaché à l'équipement de mesure (100) à relier à l'appareil de gestion (1), et
la partie de transmission-réception de données (12) communique avec le dispositif de communication (110) par l'utilisation de l'information d'identification acceptée du dispositif de communication (110), et obtient une information d'identification de l'équipement de mesure (100) par l'intermédiaire du dispositif de communication (110), dans lequel l'information d'identification est spécifique à l'équipement de mesure et est contenue dans l'équipement de mesure,
l'appareil de gestion (1) comprenant en outre une partie d'acceptation d'information d'utilisateur (13) et une partie de stockage (15),
**caractérisé en ce que**
la partie d'acceptation d'information d'utilisateur (13) accepte, en tant qu'entrée, une information relative à un utilisateur de l'équipement de mesure (100), et
la partie de stockage (15) contient l'information relative à l'utilisateur et l'information d'identification du dispositif de communication (110), et l'information d'identification de l'équipement de mesure (100) en association les unes avec les autres.

2. Appareil de gestion (1) selon la revendication 1, comprenant en outre une partie de traitement de données (14),
la partie de traitement de données (14) génère une chaîne de caractères comprenant l'information relative à l'utilisateur et l'information d'identification de l'équipement de mesure (100), en tant que nom indiquant l'équipement de mesure (100).

3. Appareil de gestion (1) selon la revendication 2, dans lequel l'information relative à l'utilisateur est un nom identifiant l'utilisateur.

4. Appareil de gestion (1) selon l'une quelconque des revendications 1 à 3, comprenant en outre :
une partie de création d'image d'affichage (16) ;
une partie d'affichage (121) ; et
une partie d'entrée (122),
dans lequel
la partie de création d'image d'affichage (16) fonctionne pour afficher, sur la partie d'affichage (121), les informations d'identification d'un ou plusieurs dispositifs de communication (110) incluant l'information d'identification du dispositif de communication (110) à entrer, et les informations d'identification d'un ou plusieurs dispositifs de communication (110) affichées sur la partie d'affichage (121) sont affichées pour être sélectionnables par l'intermédiaire de la partie d'entrée (122), et
la partie d'acceptation de données (11) fonctionne pour accepter l'information d'identification du dispositif de communication (110) sélectionnée par l'intermédiaire de la partie d'entrée (122) en tant qu'entrée de l'information d'identification du dispositif de communication (110).
